# EUROPEAN PATENT SPECIFICATION

(11) **EP 3 962 489 B1**
(45) Date of publication and mention of the grant of the patent: **22.03.2023**
(21) Application number: 20712754.9
(22) Date of filing: 20.02.2020
(51) Int. Cl.: A61K 31/567, A61K 47/34, A61P 15/18

(54) **DRUG DELIVERY SILICONE COMPOSITON TO IMPROVE ACTIVE INGREDIENT ELUTION**
ARZNEIMITTELABGABESILIKONZUSAMMENSETZUNG ZUR VERBESSERUNG DER WIRKSTOFFELUTION
COMPOSITION DE SILICONE D'ADMINISTRATION DE MÉDICAMENT DESTINÉE À AMÉLIORER L'ÉLUTION DE PRINCIPE ACTIF

(30) Priority: 22.02.2019 US 201962809311 P
(43) Date of publication of application: 09.03.2022
(73) Proprietor: Elkem Silicones USA Corp., East Brunswick, NJ 08816 (US); Elkem Silicones Germany GmbH, 23566 Lübeck (DE)
(72) Inventor: MARTIN, Francois, 23552 Lübeck (DE); BROWN, Leeanne, Chester, SC 29706 (US); KIHARA, Matthew, Dublin, CA 94568 (US); MCMULLEN, Nicole, Rock Hill, SC 29730 (US)
(74) Representative: Menville, Laure
(86) International application number: PCT/US2020/019055
(87) International publication number: WO 2020/172418

(56) References cited:
- EP-A1- 0 588 564
- EP-A1- 2 716 685
- US-A1- 2015 073 059
- US-A1- 2016 317 427

## Description

### Background

The invention relates to a new curable liquid silicone rubber composition which after curing provides a silicone elastomer useful as a drug delivery device comprising an active pharmaceutical ingredient (API) having terminal alkene, alkyne or carbonyl functionalities exhibiting an increased recovery of the active pharmaceutical ingredient.

Curable Liquid silicone rubber compositions typically cure or react to provide cured silicone rubbers, also referred to as silicone elastomers. The terms silicone rubber and silicone elastomer may be used interchangeably. Curable LSR compositions have been used since approximately 1980 and have become the raw materials of choice for manufacturing silicone rubber parts and silicone products for a wide range of applications such as medical, automotive, infant care, general industrial markets, aerospace, electronics, and many other specialized industries. Silicone rubber has been used in a wide range of biomedical applications due to their physiological inertness, high blood compatibility, low toxicity, good thermal and oxidative stability, and low modulus properties.

Silicone elastomers are strengthened with the addition of silica and/or resin in the curable liquid silicone rubber composition inducing hydrogen bonding within the composition prior curing. However, despite the ability to form hydrogen bonds, cured silicone rubbers are considered hydrophobic in nature which is mainly due to methyl constituency of siloxane polymer backbones which makes it an ideal material for solubilizing pharmaceutical agents having mostly nonpolar structures with alcohol or ketone structures. Furthermore, microporous structure makes it a material of choice for many drug delivery applications.

The concept of controlled drug release from polymeric materials was first established in the 1960s when exposure of anesthetic gases to the external surface of a silicone rubber arteriovenous shunt containing circulating rabbit blood caused the rabbits to fall asleep. Drug release occurs by diffusion through the polymer matrix. The first commercial silicone elastomer device for controlled release was Population Council's subdermal contraceptive implant Norplant^{®} marketed in the USA in 1991. It consists of six silicone elastomer cylinders, each filled internally with a therapeutically effective amount of levonorgestrel (LNG) used in many birth-control pills. While addition cure silicone elastomers are preferred for drug delivery applications due to their biostability and inert nature, certain active ingredients, such as LNG, are known to inhibit the addition cure reaction and have shown a tendency towards irreversible drug binding within the matrix. It has been established in the following reference: Boyd, P. etal., (2016) International Journal of Pharmaceutics, 511(1), 619-629, that a contraceptive ring manufactured with micronized LNG mixed in a polyaddition silicone curable composition and which is cured for 90 seconds at 160°C showed zero in vitro release of the LNG component. Some of the existing solutions to this problem are to carefully monitor the particle size of the active ingredient as well as control the elastomer's cure temperature and time or to impregnate the active pharmaceutical ingredient (API) into a fully vulcanized silicone by immersion/impregnation.

Therefore, there is still the need to find new curable liquid silicone rubber compositions that will avoid the above described problems.

Document US 2015/073059 discloses hydrosilylation curable silicone compositions that can be used for the release of active substances such as estradiol and comprise isopropyl myristate as a diluent. Document EP 0 588 564 also discloses silicone rubbers that can be used to release active ingredients such as estradiol or progesterone. Document US 2016/317427 has a similar disclosure and shows examples of silicone compositions that are cured via hydrosilylation and where isopropyl myristate is added.

### Summary

One object of the present invention is to provide a new curable liquid silicone rubber composition which after curing provides a silicone elastomer useful as a drug delivery device comprising an active pharmaceutical ingredient (API) having terminal alkene, alkyne or carbonyl functionalities exhibiting an increased recovery of the active pharmaceutical ingredient.

Another object of the invention is to provide a molded silicone rubber obtained by curing and molding the curable liquid silicone rubber composition of the invention.

Another object of the invention is to provide a drug delivery device comprising said molded silicone rubber.

### Detailed Description

All these objectives, among others, are achieved by the present invention, which relates to a curable liquid silicone rubber composition comprising:
(A) at least one organopolysiloxane **A** having at least two silicon-bonded alkenyl groups per molecule, said alkenyl groups each containing from 2 to 14 carbon atoms, preferably said alkenyl groups are chosen from the group consisting of vinyl, allyl, hexenyl, decenyl and tetradecenyl, and most preferably said alkenyl groups are vinyl groups, and
(B) at least one organosilicon compound **B** having at least two and preferably at least three silicon-bonded hydrogen atoms per molecule,
(C) at least one hydrosilylation catalyst **C,** and preferably at least one platinum-group metal catalyst **C,** and even more preferably hydrosilylation catalyst **C** is present in an amount such that the concentration is from 0.1 to 500 parts by weight of platinum-group metal, per million parts (ppm), based on the combined weight of ingredients (A) and (B),
(D) eventually at least one filler **D,** and preferably the filler **D** is a surface treated silica, and even more preferably the filler **D** is a fumed silica *in situ* treated with hexamethyldisilazane and/or tetramethyldivinyldisilazane,
(E) from 0.01% to 10 % by weight based on total weight of the curable liquid silicone rubber composition of a mixture **M** comprising at least one fatty acid ester **G** and at least one triazole compound **E,** and
(F) a therapeutically effective amount of at least one active pharmaceutical ingredient **F** comprising in its chemical structure at least one terminal alkene, at least one terminal alkyne or at least one terminal carbonyl,
   and wherein components (A) and (B) are present in an amount such that the molar ratio of silicon-bonded hydrogen atoms contained in components (B) to silicon-bonded alkenyl groups contained in component (A) ranges from 0.1 to 20, and preferably ranges from 0.25 to 5.0, and most preferably from 0.25 to 2.0. and
(G) at least one cure rate controller **G.**

To achieve this objective, the Applicant demonstrated, to its credit, entirely surprisingly and unexpectedly, that the use in a curable liquid silicone rubber composition comprising an active pharmaceutical ingredient (API) having terminal alkene, alkyne or carbonyl functionalities of a specific mixture comprising a triazole compound **E** dissolved in a fatty acid ester **G** allows to prepare after curing a silicone elastomer exhibiting an increased recovery of the active pharmaceutical ingredient by limiting the drug from complexing with platinum and from binding into the elastomeric network of the cured silicone rubber material.

In a preferred embodiment, the mixture **M** contains from 0.1% to 10% by weight of triazole compound **E** and from 99.9 to 90% by weight of fatty acid ester **G.**

In another preferred embodiment, said fatty acid ester **G** is formed from a fatty acid comprising from 2 to 20 carbon atoms. Said fatty acid is preferably selected from the group consisting of caproic acid, lauric acid, myristic acid, oleic acid, linoleic acid, adipic acid and lanolin acid.

Said fatty acid ester **G** may also be formed from an alcohol of 2 to 20 carbon atoms, and preferably said alcohol is an alkanol of 2 to 4 carbon atoms.

In another preferred embodiment, fatty acid ester **G** is isopropyl myristate.

The preferred active pharmaceutical ingredient is chosen from the group consisting of levonorgestrel, ethynyl estradiol, norethisterone, ethynodiol diacetate, desogestrel, lynestrenol, progesterone and mixtures thereof.

Component (A) is an organopolysiloxane **A** having at least two silicon-bonded alkenyl groups per molecule, said alkenyl groups each containing from 2 to 14 carbon atoms, preferably said alkenyl groups are chosen from the group consisting of vinyl, allyl, hexenyl, decenyl and tetradecenyl, and most preferably said alkenyl groups are vinyl groups.

Such component additionally comprises silicon-bonded organic groups other than alkenyl radicals. Such silicon-bonded organic groups are typically selected from monovalent saturated hydrocarbon radicals, which typically contain from 1 to 10 carbon atoms, and monovalent aromatic hydrocarbon radicals, which typically contain from 6 to 12 carbon atoms, which are unsubstituted or substituted with the groups that do not interfere with curing reaction, such as halogen atoms. Preferred species of the silicon-bonded organic groups are, for example, alkyl groups such as methyl, ethyl, and propyl; halogenated alkyl groups such as 3,3,3-trifluoropropyl; and aryl groups such as phenyl.

The molecular structure of component (A) is typically linear, however, there can be some branching due to the presence of trivalent or tertravalent siloxane units within the molecule. Preferably, the organopolysiloxane **A** has a dynamic viscosity of between 50 and 300 000 mPa.s at 25°C. All viscosities in the present disclosure are contemplated corresponds to the so-called "Newton" the amount of dynamic viscosity at 25 ° C, i.e. such that the viscosity of the measured velocity gradient is sufficiently independent of the velocity gradient at low shear using a Brookfield Viscosity dynamic viscosity meter in a manner known per se measured. The dynamic viscosity of the silicones is measured at 25°C according to the ASTM D 445 standard.

Examples of suitable organopolysiloxane **A** according to the invention are polymers of the following formula (1): 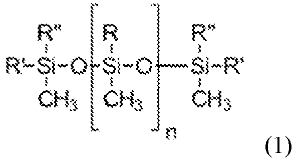 in which: R and R", are chosen independently of one another and are monovalent saturated hydrocarbon radicals, which typically contain from 1 to 10 carbon atoms, or monovalent aromatic hydrocarbon radicals, which typically contain from 6 to 12 carbon atoms, which are unsubstituted or substituted with groups that do not interfere with curing reaction, such as halogen atoms. Preferred species of the silicon-bonded organic groups are, for example, alkyl groups such as methyl, ethyl, and propyl; halogenated alkyl groups such as 3,3,3-trifluoropropyl; and aryl groups such as phenyl. R' are alkenyl groups each containing from 2 to 14 carbon atoms, preferably said alkenyl groups are chosen from the group consisting of vinyl, allyl, hexenyl, decenyl and tetradecenyl, and most preferably said alkenyl groups are vinyl groups, and most preferably R' is a vinyl radical and n represents a degree of polymerization suitable for component (A) to have a dynamic viscosity of between 50 and 300 000 mPa.s at 25°C.

As other examples of organopolysiloxane A that are of use, mention may be made of: dimethylvinylsiloxy-terminated polydimethylsiloxane, dimethylvinylsiloxy-terminated polymethyl-3,3,3-trifluoropropylslioxane, dimethylvinylsiloxy-terminated dimethylsiloxane-3,3,3-trifluoropropylmethylsiloxne copolymer, and dimethylvinylsiloxy-terminated dimethylsiloxane/methylphenylsiloxane copolymer.

Generally, component (A) has a viscosity of at least 0.05 Pa.s at 25 °C, typically from 0.1 to 300 Pa.s. more typically from 0.1 to 110 Pa.s at 25 °C.

Component (A) may also encompass silicone resins bearing Si-alkenyl groups. Examples of suitable silicone resins bearing Si-alkenyl groups comprise:
- an organopolysiloxane resin of formula MT^{Vi}Q consisting essentially of:
   - (a) trivalent siloxane units T^{Vi} of the formula R'SiO_{3/2};
   - (b) monovalent siloxane units M of the formula R₃SiO_{1/2}, and
   - (c) tetravalent siloxane units Q of the formula SiO_{4/2}
- an organopolysiloxane resin of formula MD^{Vi}Q consisting essentially of:
   - (a) divalent siloxane units D^{Vi} of the formula RR'SiO_{2/2};
   - (b) monovalent siloxane units M of the formula R₃SiO_{1/2}, and
   - (c) tetravalent siloxane units Q of the formula SiO_{4/2}
- an organopolysiloxane resin of formula MDD^{Vi}Q consisting essentially of:
   - (a) divalent siloxane units D^{Vi} of the formula RR'SiO_{2/2};
   - (b) divalent siloxane units D of the formula R₂SiO_{2/2}
   - (b) monovalent siloxane units M of the formula R₃SiO_{1/2}, and
   - (c) tetravalent siloxane units Q of the formula SiO_{4/2}
- an organopolysiloxane resin of formula M^{Vi}Q consisting essentially of:
   - (a) monovalent siloxane units M^{Vi} of the formula R'R₂SiO_{1/2}; and
   - (b) tetravalent siloxane units Q of the formula SiO_{4/2}, and
- an organopolysiloxane resin of formula M^{Vi}T^{Vi}Q consisting essentially of:
   - (a) monovalent siloxane units M^{Vi} of the formula R'R₂SiO_{1/2};
   - (b) trivalent siloxane units T^{Vi} of the formula R'SiO_{3/2}, and
   - (c) tetravalent siloxane units Q of the formula SiO_{4/2}
wherein R denotes a methyl group and R' denotes a vinyl group:

The most preferred silicone resins bearing Si-alkenyl groups is the organopolysiloxane resin of formula MD^{Vi}Q.

Component (A) may be present as a mixture of linear organopolysiloxanes bearing alkenyl groups and silicone resins bearing Si-alkenyl groups, both as described above.

Component (B) is an organosilicon compound **B** having at least two and preferably at least three silicon-bonded hydrogen atoms per molecule. The molecular configuration of organosilicon compound **B** is not specifically restricted, and it can be straight chain, branch-containing straight chain, or cyclic.

In a preferred embodiment, said organosilicon compound **B** is an organopolysiloxane comprising:
- at least three siloxy units of formula **(XL-1):**

   (H)(L)ₑ SiO_{(3-e)/2} **(XL-1)**

   in which the symbol H represents a hydrogen atom, the symbol L represents an
   alkyl having from 1 to 8 carbon atoms inclusive or a C₆ to C₁₀ aryl, and the symbol e is equal to 0, 1 or 2; and
- optionally other siloxy units of formula **(XL-2):**

   (L)_{g} SiO_{(4-g)/2} **(XL-2)**

   in which the symbol L represents an alkyl having from 1 to 8 carbon atoms inclusive or a C₆ to C₁₀ aryl and the symbol g is equal to 0, 1, 2 or 3.

The organosilicon compound **B** may be formed solely from siloxyl units of formula **(XL-1)** or may also comprise units of formula **(XL-2).** It may have a linear, branched or cyclic structure. The degree of polymerization is preferably greater than or equal to 2. More generally, it is less than 1000. Its dynamic viscosity is usually ranging from about 1 to 2000 mPa.s at 25°C, generally from about 5 to 2000 mPa.s at 25°C, or preferably from 5 to 500 mPa.s at 25°C.

Examples of suitable organosilicon compound **B** include but are not limited to:
(i) trimethylsiloxy-terminated methylhydrogenpolysiloxane,
(ii) trimethylsiloxy-terminated polydimethylsiloxane-methylhydrogensiloxane,
(iii) dimethylhydrogensiloxy-terminated dimethylsiloxane-methylhydrogensiloxane copolymers,
(iv) dimethylsiloxane-methylhydrogensiloxane cyclic copolymers,
(v) silicone resins M'Q comprising: (H)(CH₃)₂SiO_{1/2} units (M' unit) and SiO_{4/2} units (Q units), and
(vi) silicone resins MM'Q comprising: (CH₃)₃SiO_{1/2} units (M units), (CH₃)₂HSiO_{1/2} units and SiO_{4/2} units.

Component (B) may be present as a mixture of linear organopolysiloxanes bearing SiH groups and silicone resins bearing Si-H, both as described above.

Examples of suitable hydrosilylation catalysts C include hydrosilylation catalysts such as Karstedt's catalyst shown in U. S. Pat. No. 3,715,334 or other platinum or rhodium catalysts known to those in the art, and also including microencapsulated hydrosilylation catalysts for example those known in the art such as seen in U. S. Pat. No. 5,009,957. However, hydrosilylation catalysts pertinent to this invention can contain at least one of the following elements: Pt, Rh, Ru, Pd, Ni, e.g. Raney Nickel, and their combinations. The catalyst is optionally coupled to an inert or active support. Examples of preferred catalysts which can be used include platinum type catalysts such as chloroplatinic acid, alcohol solutions of chloroplatinic acid, complexes of platinum and olefins, complexes of platinum and 1,3-divinyl-1,1,3,3-tetramethyldisiloxane (known as Karstedt catalyst) and powders on which platinum is supported, etc. The platinum catalysts are fully described in the literature. Mention may in particular be made of the complexes of platinum and of an organic product described in U.S. Pat. Nos. 3,159,601, 3,159,602 and 3,220,972 and European Patents EP-A-057,459, EP-188,978 and EP-A-190,530 and the complexes of platinum and of vinylated organopolysiloxane described in US. Pat. Nos. 3,419,593, 3,715,334, 3,377,432, 3,814,730, and 3,775,452. In particular, platinum type catalysts are especially desirable. The platinum catalyst ought preferably to be used in a catalytically sufficient amount, to allow sufficiently rapid crosslinking at room temperature.

To achieve optimum physical properties that characterize some types of cured elastomer that can be prepared using the curable LSR composition of the present invention, it may be desirable to include a filler such as finely divided silica. Silica and other reinforcing fillers are often treated with one or more known filler treating agents to prevent a phenomenon referred to as "creping" or "crepe hardening" during processing of the curable composition. Typically, the filler is surface treated using for example with a fatty acid or a fatty acid ester such as a stearate, or with organosilanes, polydiorganosiloxanes, or organosilazanes hexaalkyl disilazane or short chain siloxane diols to render the filler(s) hydrophobic and therefore easier to handle and obtain a homogeneous mixture with the other ingredients.

Colloidal silicas are particularly preferred because of their relatively high surface area, which is typically at least 50 m² per gram. Colloidal silicas may be provided either as fumed silica or as a precipitated silica that may have been surfaced treated. In one method of surface treatment, the fumed silica or precipitated silica is exposed to cyclic organopolysiloxanes under heat and pressure. An additional method of treating fillers is one in which the silica is exposed to siloxanes or silanes in the presence of an amine compound.

Another method of surface treating silica fillers employs methyl silane or silazane surface treating agents. Methylsilane or silazane surface treated fumed or precipitated silica fillers exhibit the property of producing pumpable silicone compounds and also do not overly increase the low viscosity of the uncured liquid precursor silicone composition. After curing, silazane treated silicas impart an improved tear strength to the cured elastomer. U.S. Patent Nos. 3,365,743 and 3,847,848 disclose such methods.

More preferred silica fillers are *in situ* formed fumed silica with a surface area between about 50 m² per gram to about 600 m² per gram, and most preferably between about 100 m² per gram to about 400 m² per gram measured in accordance with the BET method. *In situ* treated fumed silica occurs when the silanols on the surface of the fumed silica are capped with a silicon atom containing alkyl, aryl, or alkenyl pendant groups while being compounded with the polymer in the mixer. This process can utilize hexamethyldisilazane, tetramethyldivinyldisilazane or a suitable silanol capping agent known in the art, such as trimethylsilanol and dimethylvinylsilanol to treat the filler.

The amount of finely divided silica or other reinforcing filler used in the curable LSR composition of the present invention is at least in part determined by the physical properties desired in the cured elastomer. The curable LSR composition of the present invention typically comprises from 5 to 100 parts, typically from 10 to 60 parts by weight of a reinforcing filler for every 100 parts of component (A).

Another example of a suitable filler is hydrophobic silica aerogel which is a nanostructured material with high specific surface area, high porosity, low density, low dielectric constant and excellent heat insulation properties. Silica aerogels are synthesized either via supercritical drying process or via ambient pressure drying technique so as to obtain porous structure. It is now widely commercially available.

Hydrophobic silica aerogel is characterized by a surface area ranging of from 500 to 1500 m²/g, alternatively of from 500 to 1200 m²/g, in each case determined via the BET method. The hydrophobic silica aerogel may further be characterized by its porosity above 80 %, alternatively above 90%. Hydrophobic silica aerogel may have an average particle size ranging from 5 to 1000 µm, alternatively of from 5 to 100 µm, alternatively of from 5 to 25 µm as measured by means of laser light scattering. An example of hydrophobic silica aerogel is a trimethyl silylated aerogel. The hydrophobic silica aerogel maybe presents in the curable liquid silicone rubber composition in an amount of from 1 to 30 % weight relative to the total weight of the curable liquid silicone rubber.

The cure rate controller **G,** which is also known as catalyst inhibitor, is designed to slow the curing reaction. Cure rate controllers are well known in the art and examples of such materials can be found in U.S. Patents. U.S. Patent 3,923,705 refers to the use of vinyl contained cyclic siloxanes. U.S. Patent 3,445,420 describes the use of acetylenic alcohols. U.S Patent 3,188,299 shows the effectiveness of heterocyclic amines. U.S. Patent 4,256,870 describes alkyl maleates used to control cure. Olefinic siloxanes can also be used as described in U.S. Patent 3,989,667. Polydiorganosiloxanes containing vinyl radicals have also been used and this art can be seen in U.S. Patents 3.498,945, 4,256,870, and 4,347, 346. Preferred inhibitors for this composition are methylvinylcyclosiloxanes, 3-methyl-1-butyn-3-ol, and 1-ethynyl-1-cyclohexanol with the most preferred being the 1,3,5,7-tetramethyl-1,3,5,7-tetravinyl-cyclotetrasiloxane in amounts from 0.002% to 1.00% of the silicone compound depending on the cure rate desired. The preferred cure rate controller G is chosen among: 1,3,5,7-tetramethyl-1,3,5,7-tetravinyl-cyclotetrasiloxane; 3-methyl-1-butyn-3-ol, and 1-ethynyl-1-cyclohexanol (ECH).

To obtain a longer working time or "pot life", the quantity of the cure rate controller **G1** is adjusted to reach the desired "pot life". The concentration of the catalyst inhibitor in the present silicone composition is sufficient to retard curing of the composition at ambient temperature without preventing or excessively prolonging cure at elevated temperatures. This concentration will vary widely depending on the particular inhibitor used, the nature and concentration of the hydrosilylation catalyst, and the nature of the organohydrogenopolysiloxane. Inhibitor concentrations as low as one mole of inhibitor per mole of platinum group metal will in some instances yield a satisfactory storage stability and cure rate. In other instances, inhibitor concentrations of up to 500 or more moles of inhibitor per mole of platinum group metal may be required. The optimum concentration for a particular inhibitor in a given silicone composition can be readily determined by routine experimentation.

The curable LSR composition according to the invention may further contain a chain extender which maybe be a diorganohydrogensiloxy-terminated polyorganosiloxane. As examples of suitable diorganohydrogensiloxy-terminated polyorganosiloxane, mention may be made of polydimethylsiloxanes comprising dimethyl hydrogensiloxy end groups having a dynamic viscosity at 25°C of between 1 mPa.s and 500 mPa.s, preferably of between 5 mPa.s and 200 mPa.s, even more preferentially of between 1 and 30 mPa.s.

Particularly advantageous chain extender are poly(dimethylsiloxy)-α,ω-(dimethyl hydrogensiloxy) of formula M^{H}DₓM^{H} in which:
- M^{H} = siloxy unit of formula: (H)(CH₃)₂SiO_{1/2}
- D = siloxy unit of formula: (CH₃)₂SiO_{2/2}, and
- x is an integer which value is between 1 and 200, preferably between 1 and 150 and even more preferentially between 3 and 120.

The chain extender is described as "chain extender" since it has the presumed effect of increasing the mesh size of the network when it is crosslinked. When the SiH reactive functions are at the chain end, the term "telechelic" polymer is sometimes used.

The curable liquid rubber composition according to the invention may be store as a two-part component system prior use (Part-A and Part-B). The first component (Part-A) may comprise the vinyl siloxane polymer(s) (component (A)), the filler(s) **D** and the hydrosilylation catalyst **C,** whereas the second component (part-B) may comprise the vinyl siloxane polymer(s) (component (A)), the filler(s) **D,** the organosilicon compound **B,** the cure rate controller **G** and the mixture **M** comprising at least one fatty acid ester **G** and at least one triazole compound E, whereas said therapeutically effective amount of active pharmaceutical ingredient **F** may be stored in a different part and added just prior curing to a mixture prepared by mixing the components of part-A and part-B (1:1 weight ratio) to prepare the curable liquid silicone rubber composition according to the invention and as described above.

Curing of the curable liquid silicone rubber composition may be carried at as required by the type of liquid silicone rubber utilized. Typical curing temperatures may range of from 60°C to 220°C, alternatively of from 80°C to 190°C.

In one embodiment, the present invention relates to a molded silicone rubber obtained by curing and molding said curable liquid silicone rubber composition as defined above and according to the invention, preferably by heating and molding at a temperature ranging from 60°C to up to 220°C.

Curing can for example take place in a mold to form a molded silicone article. The curable liquid silicone rubber composition according to the invention may for example be cured and molded to prepare a drug delivery device, and preferably via an injection molding apparatus or a compression molding apparatus.

In another embodiment, the invention concerns a drug delivery device as described above characterized in that it is an intravaginal drug delivery device.

The present invention will now be disclosed by means of the following nonlimiting examples.

### EXAMPLES

### I) Raw materials

- Organopolysiloxane A-1 = polydimethylsiloxane with dimethylvinylsilyl end-units with a viscosity at 25°C of about 4000 mPa.s;
- Organopolysiloxane A-2 = polydimethylsiloxane with dimethylvinylsilyl end-units with a viscosity at 25°C of about 100 mPa.s;
- Organopolysiloxane resin mixture 1 consisting of:
   - 60% by weight of organopolysiloxane A-3 = polydimethylsiloxane with dimethylvinylsilyl end-units with a viscosity at 25°C of about 60,000 mPa.s A-3, and
   - 40% by weight of silicone resin of formula MD^{Vi}Q consisting essentially of:
      - (a) divalent siloxane units D^{Vi} of the formula RR'SiO_{2/2};
      - (b) monovalent siloxane units M of the formula R₃SiO_{1/2},
      - (c) tetravalent siloxane units Q of the formula SiO_{4/2} , and
      wherein R denotes a methyl group and R' denotes a vinyl group
- LSR base 1 contains a mixture of:
   - organopolysiloxane A-4 = polydimethylsiloxane with dimethylvinylsilyl end-units with a viscosity at 25°C of about 100,000 mPa.s;
   - organopolysiloxane A-3 = polydimethylsiloxane with dimethylvinylsilyl end-units with a viscosity at 25°C of about 60,000 mPa.s; and
   - around 30% by weight of silica having a BET specific surface area of around 300 m²/g and situ treated by hexamethyldisilazane and tetramethyldivinylsilazane.
- LSR base 2 contains a mixture of:
   - organopolysiloxane A-5 = polydimethylsiloxane with dimethylvinylsilyl end-units with a viscosity at 25°C of about 20,000 mPa.s; and
   - around 30% by weight of silica having a BET specific surface area of around 300 m²/g and situ surface treated by hexamethyldisilazane and tetramethyldivinylsilazane.
- Organosilicon compound **B-1:** silicone resins (M'Q) comprising: (H)(CH₃)₂SiO_{1/2} units and SiO_{4/2} units.
- Mixture M1= 1% by weight of benzotriazole and 99% by weight of isopropyl myristate.
- Catalyst C1-= 10% by weight of Platinum metal, known as Karstedt's catalyst diluted in a dimethylvinyldimer (350 m.Pa.s) and sold by Johnson Matthey Company;
- Cure rate controller **G-1:** 1-Ethynyl-1-cyclohexanol (ECH)

**Table 1: Formulations**

| **Compositions** | 1 | 2 | 3 | 4 | 5 | 6 | 7 | 8 |
|---|---|---|---|---|---|---|---|---|
| **Components of Part-A** | %by weight | % by weight | % by weight | % by weight | % by weight | % by weight | % by weight | % by weight |
| LSR base 1 | 84.97 | 91.50 | 84.97 | 0 | 84.97 | 0 | 0 | 0 |
| LSR base 2 | 0 | 0 | 0 | 84.97 | 0 | 84.97 | 84.97 | 92.97 |
| Organopolysiloxane A-1 | 14.99 | 14.46 | 0 | 14.99 | 0 | 14.99 | 14.99 | 6.99 |
| Organopolysiloxane A-2 | 0 | 0 | 14.99 | 0 | 0 | 0 | 0 | 0 |
| Organopolysiloxane resin mixture 1 | 0 | 0 | 0 | 0 | 14.99 | 0 | 0 | 0 |
| Catalyst C1 | 0.04 | 0.04 | 0.04 | 0.04 | 0.04 | 0.04 | 0.04 | 0.04 |

| **Components of Part-B** | | | | | | | | |
|---|---|---|---|---|---|---|---|---|
| LSR base 1 | 83.13 | 90.92 | 83.37 | 0 | 83.13 | 0 | 0 | 0 |
| LSR base 2 | 0 | 0 | 0 | 82.90 | 0 | 84.97 | 83.37 | 87.37 |
| Organopolysiloxane A-1 | 14.67 | 7.01 | 0 | 12.77 | 0 | 12.35 | 0 | 0 |
| Organopolysiloxane A-2 | 0 | 0 | 12.40 | 0 | 0 | 0 | 12.40 | 8.30 |
| Organopolysiloxane resin mixture 1 | 0 | 0 | 0 | 0 | 13.30 | 0 | 0 | 0 |
| Organosilicon compound B-1 | 1.37 | 1.24 | 3.40 | 3.50 | 2.74 | 1.85 | 3.40 | 3.50 |
| Mixture M1 | 0.59 | 0.59 | 0.59 | 0.59 | 0.59 | 0.59 | 0.59 | 0.59 |
| Cure rate controller G-1 | 0.24 | 0.24 | 0.24 | 0.24 | 0.24 | 0.24 | 0.24 | 0.24 |

Curable liquid silicone rubber compositions 1 to 8 were prepared by mixing on 1:1 weight ratio Part-A and Part-B. Then an active pharmaceutical ingredient (levonorgestrel) was added at various amount prior to injection molding (curing at a temperature of around 180°C) to yield a molded silicone rubber suitable as a drug delivery device.

## Claims

1. A curable liquid silicone rubber composition comprising:
(A) at least one organopolysiloxane **A** having at least two silicon-bonded alkenyl groups per molecule, said alkenyl groups each containing from 2 to 14 carbon atoms, preferably said alkenyl groups are chosen from the group consisting of vinyl, allyl, hexenyl, decenyl and tetradecenyl, and most preferably said alkenyl groups are vinyl groups, and
(B) at least one organosilicon compound **B** having at least two and preferably at least three silicon-bonded hydrogen atoms per molecule,
(C) at least one hydrosilylation catalyst **C,** and preferably at least one platinum-group metal catalyst **C,** and even more preferably hydrosilylation catalyst **C** is present in an amount such that the concentration is from 0.1 to 500 parts by weight of platinum-group metal, per million parts (ppm), based on the combined weight of ingredients (A) and (B),
(D) eventually at least one filler **D,** and preferably the filler **D** is a surface treated silica, and even more preferably the filler **D** is a fumed silica *in situ* treated with hexamethyldisilazane and/or tetramethyldivinyldisilazane,
(E) from 0.01% to 10 % by weight based on total weight of the curable liquid silicone rubber composition of a mixture **M** comprising at least one fatty acid ester **G** and at least one triazole compound **E,**
(F) a therapeutically effective amount of at least one active pharmaceutical ingredient **F** comprising in its chemical structure at least one terminal alkene, at least one terminal alkyne or at least one terminal carbonyl,
and wherein components (A) and (B) are present in an amount such that the molar ratio of silicon-bonded hydrogen atoms contained in components (B) to silicon-bonded alkenyl groups contained in component (A) ranges from 0.1 to 20, and preferably ranges from 0.25 to 5.0, and most preferably from 0.25 to 2.0, and
(G) at least one cure rate controller **G.**

2. A curable liquid silicone rubber composition according to claim 1 wherein the mixture **M** contains from 0.1% to 10% by weight of triazole compound **E** and from 99.9 to 90% by weight of fatty acid ester **G.**

3. A curable liquid silicone rubber composition according to claim 1 wherein said fatty acid ester **G** is formed from a fatty acid comprising from 2 to 20 carbon atoms.

4. A curable liquid silicone rubber composition according to claim 3 wherein said fatty acid is selected from the group consisting of caproic acid, lauric acid, myristic acid, oleic acid, linoleic acid, adipic acid and lanolin acid.

5. A curable liquid silicone rubber composition according to claim 1 wherein said fatty acid ester **G** is formed from an alcohol of 2 to 20 carbon atoms

6. A curable liquid silicone rubber composition according to claim 4 wherein said alcohol is an alkanol of 2 to 4 carbon atoms.

7. A curable liquid silicone rubber composition according to claim 1 wherein said fatty acid ester **G** is isopropyl myristate.

8. A curable liquid silicone rubber composition according to claim 1 wherein said active pharmaceutical ingredient is chosen from the group consisting of levonorgestrel, ethynyl estradiol, norethisterone, ethynodiol diacetate, desogestrel, lynestrenol, progesterone and mixtures thereof.

9. A molded silicone rubber obtained by curing and molding said curable liquid silicone rubber composition as defined in anyone of claims 1 to 8, preferably by heating and molding at a temperature ranging from 60°C to up to 220°C.

10. A drug delivery device comprising a molded silicone rubber according to claim 9.

11. A drug delivery device comprising a molded silicone rubber according to claim 9 prepared via an injection molding apparatus or a compression molding apparatus.

12. A drug delivery device according to claim 10 or 11 **characterized in that** it is an intravaginal drug delivery device.

## Patentansprüche

1. Härtbare flüssige Silikonkautschukzusammensetzung, umfassend:
(A) mindestens ein Organopolysiloxan **A** mit mindestens zwei siliciumgebundenen Alkenylgruppen pro Molekül, wobei die Alkenylgruppen jeweils 2 bis 14 Kohlenstoffatome enthalten, die Alkenylgruppen vorzugsweise aus der Gruppe bestehend aus Vinyl, Allyl, Hexenyl, Decenyl und Tetradecenyl ausgewählt sind und es sich bei den Alkenylgruppen ganz besonders bevorzugt um Vinylgruppen handelt, und
(B) mindestens eine Organosiliciumverbindung **B** mit mindestens zwei und vorzugsweise mindestens drei siliciumgebundenen Wasserstoffatomen pro Molekül,
(C) mindestens einen Hydrosilylierungskatalysator **C** und mindestens einen Platingruppenmetallkatalysator **C,** wobei der Hydrosilylierungskatalysator **C** noch weiter bevorzugt in einer solchen Menge vorliegt, dass die Konzentration 0,1 bis 500 Gewichtsteile Platingruppenmetall pro Million Teile (ppm), bezogen auf das kombinierte Gewicht der Bestandteile (A) und (B), beträgt,
(D) gegebenenfalls mindestens einen Füllstoff **D,** wobei es sich bei dem Füllstoff **D** vorzugsweise um oberflächenbehandeltes Siliciumdioxid handelt und es sich bei dem Füllstoff **D** noch weiter bevorzugt um ein in situ mit Hexamethyldisilazan und/oder Tetramethyldivinyldisilazan behandeltes pyrogenes Siliciumdioxid handelt,
(E) 0,01 bis 10 Gew.-%, bezogen auf das Gesamtgewicht der härtbaren flüssigen Silikonkautschukzusammensetzung, einer Mischung **M,** die mindestens einen Fettsäureester **G** und mindestens eine Triazolverbindung **E** umfasst,
(F) eine therapeutisch wirksame Menge mindestens eines pharmazeutischen Wirkstoffs **F,** der in seiner chemischen Struktur mindestens ein endständiges Alken, mindestens ein endständiges Alkin oder mindestens ein endständiges Carbonyl umfasst,
und wobei die Komponenten (A) und (B) in einer solchen Menge vorliegen, dass das Molverhältnis von in Komponente (B) enthaltenen siliciumgebundenen Wasserstoffatomen zu in Komponente (A) enthaltenen siliciumgebundenen Alkenylgruppen im Bereich von 0,1 bis 20 liegt und vorzugsweise im Bereich von 0,25 bis 5,0 und ganz besonders bevorzugt von 0,25 bis 2,0 liegt, und
(G) mindestens einen Härtungsgeschwindigkeitsregler **G.**

2. Härtbare flüssige Silikonkautschukzusammensetzung nach Anspruch 1, wobei die Mischung **M** 0,1 bis 10 Gew.-% Triazolverbindung **E** und 99,9 bis 90 Gew.-% Fettsäureester **G** enthält.

3. Härtbare flüssige Silikonkautschukzusammensetzung nach Anspruch 1, wobei der Fettsäureester **G** aus einer Fettsäure mit 2 bis 20 Kohlenstoffatomen gebildet ist.

4. Härtbare flüssige Silikonkautschukzusammensetzung nach Anspruch 3, wobei die Fettsäure aus der Gruppe bestehend aus Capronsäure, Laurinsäure, Myristinsäure, Ölsäure, Linolsäure, Adipinsäure und Lanolinsäure ausgewählt ist.

5. Härtbare flüssige Silikonkautschukzusammensetzung nach Anspruch 1, wobei der Fettsäureester **G** aus einem Alkohol mit 2 bis 20 Kohlenstoffatomen gebildet ist.

6. Härtbare flüssige Silikonkautschukzusammensetzung nach Anspruch 4, wobei es sich bei dem Alkohol um ein Alkanol mit 2 bis 4 Kohlenstoffatomen handelt.

7. Härtbare flüssige Silikonkautschukzusammensetzung nach Anspruch 1, wobei es sich bei dem Fettsäureester **G** um Isopropylmyristat handelt.

8. Härtbare flüssige Silikonkautschukzusammensetzung nach Anspruch 1, wobei der pharmazeutische Wirkstoff aus der Gruppe bestehend aus Levonorgestrel, Ethinylestradiol, Norethisteron, Ethinodioldiacetat, Desogestrel, Lynesterol, Progesteron und Mischungen davon ausgewählt ist.

9. Geformter Silikonkautschuk, erhalten durch Härten und Formen der härtbaren flüssigen Silikonkautschukzusammensetzung gemäß einem der Ansprüche 1 bis 8, vorzugsweise durch Erhitzen und Formen bei einer Temperatur im Bereich von 60 °C bis 220 °C.

10. Arzneistoffzuführungsvorrichtung, umfassend einen geformten Silikonkautschuk nach Anspruch 9.

11. Arzneistoffzuführungsvorrichtung, umfassend einen mit Hilfe einer Spritzgussmaschine oder einer Formpressmaschine geformten Silikonkautschuk nach Anspruch 9.

12. Arzneistoffzuführungsvorrichtung nach Anspruch 10 oder 11, **dadurch gekennzeichnet, dass** es sich dabei um eine intravaginale Arzneistoffzuführungsvorrichtung handelt.

## Revendications

1. Composition de caoutchouc de silicone liquide durcissable comprenant :
(A) au moins un organopolysiloxane A possédant au moins deux groupes alcényle liés au silicium par molécule, lesdits groupes alcényle contenant chacun de 2 à 14 atomes de carbone, préférablement lesdits groupes alcényle étant choisis dans le groupe constitué par vinyle, allyle, hexényle, décényle et tétradécényle, et le plus préférablement lesdits groupes alcényle étant des groupes vinyle, et
(B) au moins un composé d'organosilicium B possédant au moins deux et préférablement au moins trois atomes d'hydrogène liés au silicium par molécule,
(C) au moins un catalyseur d'hydrosilylation C, et préférablement au moins un catalyseur C de métal du groupe du platine, et encore plus préférablement un catalyseur d'hydrosilylation C étant présent en une quantité telle que la concentration va de 0,1 à 500 parties en poids de métal du groupe du platine, par million de parties (ppm), sur la base du poids combiné des ingrédients (A) et (B),
(D) éventuellement au moins une charge D, et préférablement la charge D étant une silice traitée en surface, et encore plus préférablement la charge D étant une silice fumée traitée *in situ* par de l'hexaméthyldisilazane et/ou du tétraméthyldivinyldisilazane,
(E) de 0,01 % à 10 % en poids sur la base du poids total de la composition de caoutchouc de silicone liquide durcissable d'un mélange M comprenant au moins un ester G d'acide gras et au moins un composé de type triazole E,
(F) une quantité thérapeutiquement efficace d'au moins un ingrédient pharmaceutique actif F comprenant dans sa structure chimique au moins un alcène terminal, au moins un alcyne terminal ou au moins un carbonyle terminal,
et les composants (A) et (B) étant présents en une quantité telle que le rapport molaire d'atomes d'hydrogène liés au silicium contenus dans les composants (B) sur les groupes alcényle liés au silicium contenus dans le composant (A) se situe dans la plage de 0,1 à 20, et préférablement se situe dans la plage de 0,25 à 5,0, et le plus préférablement de 0,25 à 2,0, et
(G) au moins un agent G de régulation de la vitesse de durcissement.

2. Composition de caoutchouc de silicone liquide durcissable selon la revendication 1, le mélange M contenant de 0,1 % à 10 % en poids de composé de triazole E et de 99,9 à 90 % en poids d'ester G d'acide gras.

3. Composition de caoutchouc de silicone liquide durcissable selon la revendication 1, ledit ester G d'acide gras étant formé à partir d'un acide gras comprenant de 2 à 20 atomes de carbone.

4. Composition de caoutchouc de silicone liquide durcissable selon la revendication 3, ledit acide gras étant choisi dans le groupe constitué par l'acide caproïque, l'acide laurique, l'acide myristique, l'acide oléique, l'acide linoléique, l'acide adipique et l'acide lanolique.

5. Composition de caoutchouc de silicone liquide durcissable selon la revendication 1, ledit ester G d'acide gras étant formé à partir d'un alcool de 2 à 20 atomes de carbone.

6. Composition de caoutchouc de silicone liquide durcissable selon la revendication 4, ledit alcool étant un alcanol de 2 à 4 atomes de carbone.

7. Composition de caoutchouc de silicone liquide durcissable selon la revendication 1, ledit ester G d'acide gras étant le myristate d'isopropyle.

8. Composition de caoutchouc de silicone liquide durcissable selon la revendication 1, ledit ingrédient pharmaceutique actif étant choisi dans le groupe constitué par le lévonorgestrel, l'éthynylestradiol, la noréthistérone, le diacétate d'éthynodiol, le désogestrel, le lynestrénol, la progestérone et des mélanges correspondants.

9. Caoutchouc de silicone moulée obtenu par durcissement et moulage de ladite composition de caoutchouc de silicone liquide durcissable telle que définie dans l'une quelconque des revendications 1 à 8, préférablement en chauffant et en moulant à une température se situant dans la plage de 60 °C à jusqu'à 220 °C.

10. Dispositif d'administration de médicament comprenant un caoutchouc de silicone moulée selon la revendication 9.

11. Dispositif d'administration de médicament comprenant un caoutchouc de silicone moulée selon la revendication 9 préparé via un appareil de moulage par injection ou un appareil de moulage par compression.

12. Dispositif d'administration de médicament selon la revendication 10 ou 11 **caractérisé en ce qu'**il est un dispositif d'administration de médicament intravaginal.
